# EUROPÄISCHE PATENTSCHRIFT

(11) **EP 2 435 064 B1**
(45) Veröffentlichungstag und Bekanntmachung des Hinweises auf die Patenterteilung: **19.08.2015**
(21) Anmeldenummer: 10729701.2
(22) Anmeldetag: 27.05.2010
(51) Int. Cl.: A61K 38/07, A61P 35/00, A61P 31/12, A61P 11/00

(54) **VERWENDUNG VON HEMMSTOFFEN DER HAT UND TMPRSS2 ALS ARZNEIMITTEL**
USE OF HAT INHIBITORS AND TMPRSS2 INHIBITORS AS MEDICAMENTS
UTILISATION D'INHIBITEURS DE LA HAT ET DE LA TMPRSS2 EN TANT QUE MÉDICAMENTS

(30) Priorität: 27.05.2009 DE 102009022794
(43) Veröffentlichungstag der Anmeldung: 04.04.2012
(73) Patentinhaber: Philipps-Universität Marburg, 35037 Marburg (DE)
(72) Erfinder: STEINMETZER, Torsten, 07743 Jena (DE); SIELAFF, Frank, 35041 Marburg (DE); GARTEN, Wolfgang, 35392 Gießen (DE); BÖTTCHER, Eva, 35039 Marburg (DE); FREUER, Catharina, 35037 Marburg (DE); MATROSOVICH, Mikhail, 35039 Marburg (DE); MATROSOVICH, Tatyana, 35039 Marburg (DE)
(74) Vertreter: Patentanwälte Olbricht Buchhold Keulertz
(86) Internationale Anmeldenummer: PCT/EP2010/057334
(87) Internationale Veröffentlichungsnummer: WO 2010/149459

(56) Entgegenhaltungen:
- EP-A2- 1 182 207
- WO-A2-01/96286
- WO-A2-03/076391
- DE-A1-102006 048 300
- US-B1- 6 797 504
- SCHWEINITZ ANDREA ET AL: "Design of novel and selective inhibitors of urokinase-type plasminogen activator with improved pharmacokinetic properties for use as antimetastatic agents" JOURNAL OF BIOLOGICAL CHEMISTRY, Bd. 279, Nr. 32, 6. August 2004 (2004-08-06), Seiten 33613-33622, XP002597718 ISSN: 0021-9258
- BANKE INGO J ET AL: "Increase of anti-metastatic efficacy by selectivity-but not affinity-optimization of synthetic serine protease inhibitors" BIOLOGICAL CHEMISTRY, WALTER DE GRUYTER GMBH & CO, BERLIN, DE, Bd. 384, Nr. 10-11, 1. Oktober 2003 (2003-10-01), Seiten 1515-1525, XP009137831 ISSN: 1431-6730
- ETTMAYER PETER ET AL: "Lessons learned from marketed and investigational prodrugs", JOURNAL OF MEDICINAL CHEMISTRY, vol. 47, no. 10, 6 May 2004 (2004-05-06), pages 2393-2404, ISSN: 0022-2623
- MATSUSHIMA RIE ET AL: "Human airway trypsin-like protease stimulates human bronchial fibroblast proliferation in a protease-activated receptor-2-dependent pathway", AMERICAN JOURNAL OF PHYSIOLOGY - LUNG CELLULAR AND MOLECULAR PHYSIOLOGY, vol. 290, no. 2, February 2006 (2006-02), pages L385-L395, ISSN: 1040-0605
- SIELAFF FRANK ET AL: "Development of substrate analogue inhibitors for the human airway trypsin-like protease HAT", BIOORGANIC & MEDICINAL CHEMISTRY LETTERS, vol. 21, no. 16, August 2011 (2011-08), pages 4860-4864,

## Beschreibung

Die vorliegende Erfindung beschreibt die Verwendung von Hemmstoffen der Serinproteasen HAT und TMPRSS2 zur Herstellung von Arzneimitteln, die zur Behandlung von Erkrankungen einsetzbar sind, bei denen diese Proteasen beteiligt sind. So sind Hemmstoffe beider Enzyme für die Behandlung virusbedingter Erkrankungen geeignet, beispielsweise von Influenzainfektionen. Inhibitoren der HAT sind auch für die Therapie von inflammatorischen Atemwegserkrankungen einsetzbar, während Hemmstoffe der TMPRSS2 zusätzlich für die Behandlung von Krebs und einer damit verbundenen Metastasierung verwendet werden können.

### Beschreibung und Einleitung des allgemeinen Gebietes der Erfindung

Die vorliegende Erfindung betrifft die Gebiete Pharmazie, Medizin, organische Chemie und Biochemie.

### Stand der Technik

Die vorliegende Erfindung betrifft die Verwendung von Tripeptidmimetika als Hemmstoffe der Serinproteasen HAT und TMPRSS2 zur Herstellung von Arzneimitteln, die für die Therapie und/oder Prophylaxe von viralen Erkrankungen und der Behandlung von Tumoren bzw. der Reduktion der Metastasierung eingesetzt werden können. Ebenso ist eine Verwendung dieser Verbindungen zur Therapie und/oder Prophylaxe von inflammatorischen Atemwegserkrankungen möglich.

Die HAT (auch als *human airway trypsin-like protease* oder *adrenal secretory serine protease* bezeichnet, accession code: AB002134) und die TMPRSS2 (accession code: U75329) gehören zur Gruppe der Typ II Transmembranproteine und besitzen eine trypsinartige Serinproteasedomäne. Erste Ergebnisse weisen darauf hin, dass beide Enzyme an verschiedenen Erkrankungen beteiligt sein könnten.

Die Serinprotease HAT wurde ursprünglich aus dem Sputum von Patienten mit chronischen Atemwegserkrankungen isoliert (Yasuoka et al., Am. J. Respir. Cell Mol. Biol. 16, 300-308 (1997), allerdings sind bisher nur wenige Arbeiten zur physiologischen Funktion der HAT erschienen. Es wurde aber gezeigt, dass HAT in der Lage ist, Influenza-Viren zu aktivieren, indem sie das nicht fusionsfähige HA0-Hämagglutinin auf der Virenoberfläche in HA1 und HA2 spaltet, wodurch die Viren die Fähigkeit erlangen, in Wirtszellen eindringen zu können (Böttcher et al., J. Virology 80, 9896-9898 (2006)). Für HAT wurde gefunden, dass sie möglicherweise auch wichtige Funktionen bei entzündlichen Prozessen in den Atemwegsorganen besitzt, die über die HAT-katalysierte Aktivierung des PAR-2 Rezeptors vermittelt werden (Matsushima et al., Am J. Physiol Lung Cell Mol Physiol. 290, L385-L395 (2006)). In einer anderen Arbeit wurde beschrieben, dass die HAT möglicherweise für die erhöhte Schleimproduktion bei Patienten mit chronischen Atemwegserkrankungen verantwortlich ist und dadurch das Krankheitsbild verstärkt (Chokki et al., Am. J. Respir. Cell Mol. Biol. 30 470- 478 (2004)). Möglicherweise besitzt die HAT auch bei Krebs und der Metastasierung eine wichtige Funktion, indem sie extrazelluläre Matrixproteine abbaut, aber auch Prohormone oder Zymogenformen anderer Proteasen aktivieren kann.

Das Gen für die Serinprotease TMPRSS2 wurde erstmals 1997 identifiziert (Paolini-Giacobino, Genomics 44, 309-320 (1997)). Für die TMPRSS2 wurde auch beschrieben, dass sie an viralen Erkrankungen beteiligt sein kann, beispielsweise an Influenza- oder Metapneumovirus-Infektionen (Böttcher et al., J. Virology 80, 9896-9898 (2006), Chaipan et al., J. Virology 83, 3200-3211 (2009), Shirogane et al., J. Virology 82, 8942-8946 (2008)). Für die TMPRSS2 wurde ebenfalls eine erhöhte Expression bzw. Misslokalisierung bei Krebserkrankungen beobachtet (Afar et al., Cancer Res. 61, 1686-1692 (2001), Varaala et al., Int. J. Cancer 94, 705-710 (2001), Lucas et al., J. Pathology 215, 118-125 (2008), daher wird vermutet, dass man mit Hemmstoffen der TMPRSS2 potentiell die Möglichkeit hat, Krebs und eine damit verbundene Metastasierung der Tumore behandeln zu können.

Bisher sind keine synthetischen Inhibitoren der HAT und TMPRSS2 bekannt. Der Erfindung liegt daher die Aufgabe zu Grunde, für therapeutische Anwendungen geeignete Wirkstoffe bereitzustellen, die die Serinproteasen HAT und TMPRSS2 mit hoher Aktivität inhibieren und daher für die Behandlung von Erkrankungen geeignet sind, bei denen diese Proteasen beteiligt sind.

Durch ein Screening bekannter Hemmstoffe für trypsinartige Serinproteasen, die beispielsweise bereits als Inhibitoren der Proteasen Faktor Xa, Thrombin oder Urokinase (uPA) beschrieben wurden (z.B. Schweinitz et al., J. Biol. Chem. 279, 33613-336122 (2004), Schweinitz et al., Medicinal Chemistry 2, 349-361 (2006), Stürzebecher et al., ChemMedChem 2, 1043-1053 (2007), Hellstern et al., J. Thromb. Haemost. 5, 2119-2126 (2007)), haben wir überraschenderweise Verbindungen gefunden, die die Serinproteasen HAT und TMPRSS2 wirksam hemmen und deshalb für die Behandlung von Erkrankungen einsetzbar sind, bei denen diese Proteasen beteiligt sind.

Die Aufgabe wird durch Inhibitoren der Serinproteasen HAT und TMPRSS2 gemäß Patentanspruch 1 gelöst. Als geeignet für die Verwendung bei der Therapie und/oder Prophylaxe viraler Infektionen und inflammatorischer Atemwegserkrankungen durch Inhibierung der Serinproteasen HAT und TMPRSS2 erwiesen sich demnach substratanaloge Inhibitoren der allgemeinen Formel (I), 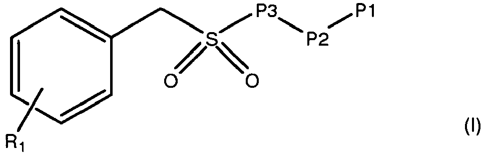 sowie die pharmazeutisch geeigneten Salze oder Prodrugs dieser Verbindungen, wobei die in der Anmeldung verwendete Bezeichnung P1, P2, und P3 der von Schechter und Berger vorgeschlagenen Nomenklatur zur Bezeichnung der Aminosäurepositionen in Proteasesubstraten und substratanalogen Inhibitoren entspricht (Schechter & Berger, Biochem. Biophys. Res. Comm., 1967, 27, 157) und worin
- R₁ sich in 2-, 3- oder 4-Stellung befindet und ausgewählt ist aus -H, oder R₂-CO-NH-CH₂-, worin R₂ eine lineare oder verzweigte Alkylgruppe mit 1 bis 9 Kohlenstoffatomen darstellt, und
- P3 eine einfach oder mehrfach substituierte oder unsubstituierte, natürliche oder unnatürliche α-Aminosäure in der D-Konfiguration ist, und
- P2 eine einfach oder mehrfach substituierte oder unsubstituierte, natürliche oder unnatürliche α-Aminosäure oder α-Iminosäure in der L-Konfiguration ist, und
- P1 ausgewählt wird aus folgenden Strukturen 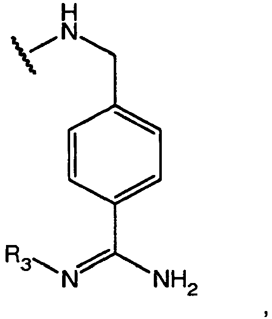 worin R₃ ein H, OH, O-CH₃, NH₂, O-CO-CH₃ oder -CO-O-(CH₂)_{z}-CH₃ ist und z eine ganze Zahl von 1 bis 5 ist;
   wobei P3 eine Struktur gemäß Formel (II) darstellt 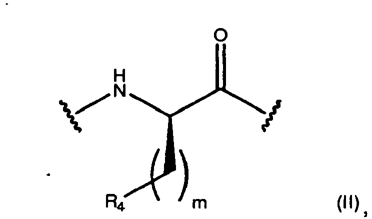 worin
- m = 0, 1, 2, 3, 4 oder 5 ist, und
- R₄ ausgewählt wird aus
   - einer unsubstituierten oder gegebenenfalls substituierten Hydroxy-, Amino-, Amid-, -S-CH₃, Amidino-, Guanidino- oder -O-Guanidinogruppe der folgenden Strukturen 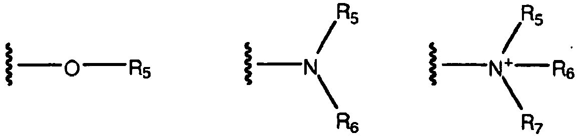 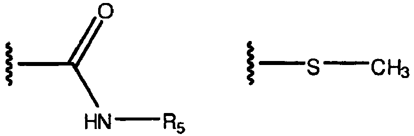 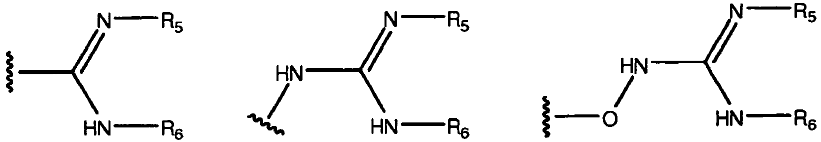 wobei R₅, R₆ und R₇ unabhängig voneinander ein H oder ein Aralkylrest sein können, oder
   - aus einem unsubstituierten oder an beliebiger Stelle substituierten Aryl-oder Heteroarylring mit 5 bis 10 Ringatomen, wobei der Heteroarylring bis zu 4 Heteroatome ausgewählt aus N, O oder S enthalten kann, und der gegebenenfalls vorhandene Substituent am Aryl- oder Heteroarylring ausgewählt wird aus Cyano, CF₃, F, Cl, Br, Amino, Aminomethylen, Amidino, Guanidino, oder aus folgenden Strukturen 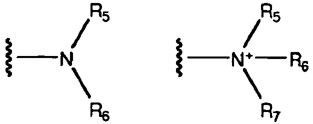 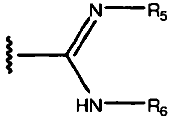 wobei die Reste R₅, R₆ und R₇ wie zuvor definiert sind, oder
   - aus einem unsubstituiertem oder an beliebiger Stelle substituierten Cycloalkylring mit 5 bis 7 Ringatomen;
      wobei P2 einen α-Aminosäure- oder α-Iminosäurerest gemäß der folgenden Strukturen darstellt 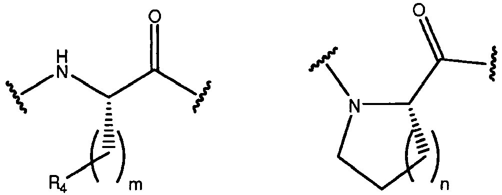 wobei n gleich 0, 1 oder 2 sein kann, und m und R₄ wie zuvor definiert sind und wenn m gleich 0 oder 1 ist, kann R₄ H sein.

Als erfindungsgemäß geeignet für die Verwendung als Hemmstoffe der HAT und TMPRSS2 und mit diesen Proteasen in Zusammenhang stehenden Erkrankungen erwiesen sich Verbindungen, die dadurch gekennzeichnet sind, dass P1 einen 4-Amidinobenzylamidrest gemäß Formel (III) darstellt 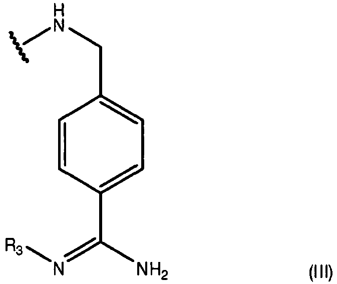 wobei R₃ wie zuvor definiert ist, bevorzugt ist R₃ ein H.

Als besonders geeignet für die Verwendung als Hemmstoffe der HAT und TMPRSS2 und für die Behandlung von Erkrankungen, die mit diesen Proteasen in Zusammenhang stehen, erwiesen sich Verbindungen, die dadurch gekennzeichnet sind, dass sich der Rest R₁ in 3- oder 4-Stellung des Phenylrings befindet, ganz besonders bevorzugt ist R₁ ein H.

Als besonders geeignet für die Verwendung als Hemmstoffe der HAT und TMPRSS2 und für die Behandlung von Erkrankungen, die mit diesen Proteasen in Zusammenhang stehen, erwiesen sich Verbindungen, die dadurch gekennzeichnet sind, dass der Rest P3 aus folgenden Strukturen ausgewählt wird 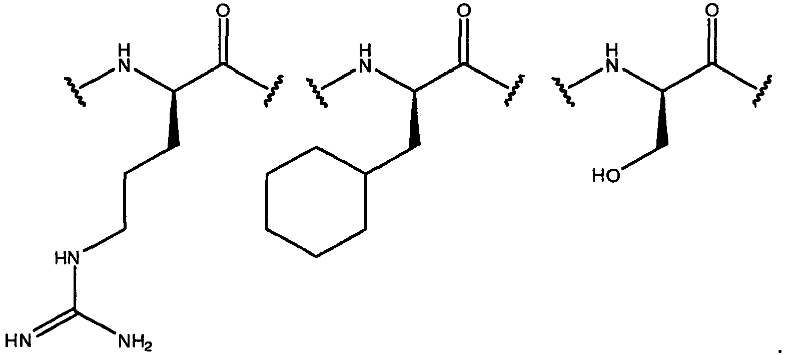 und einen D-Arginin-, D-Cyclohexylalanin oder D-Serinrest darstellt.

Als besonders geeignet für die Verwendung als Hemmstoffe der HAT und TMPRSS2 und für die Behandlung von Erkrankungen, die mit diesen Proteasen in Zusammenhang stehen, erwiesen sich Verbindungen, die dadurch gekennzeichnet sind, dass der Rest P2 aus folgenden Strukturen ausgewählt wird 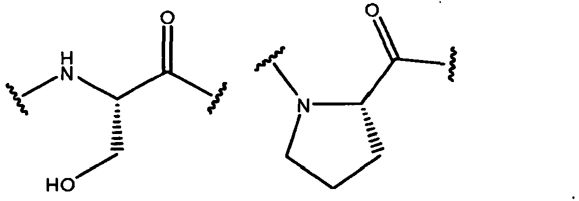 und einen Serin oder Prolinrest in L-Konfiguration darstellt.

Als besonders geeignet für die Verwendung als Hemmstoffe der HAT und TMPRSS2 und für die Behandlung von Erkrankungen, die mit diesen Proteasen in Zusammenhang stehen, erwiesen sich folgende Verbindungen

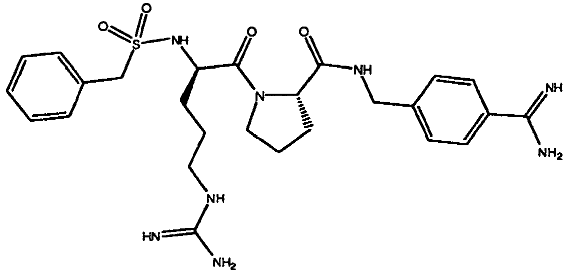

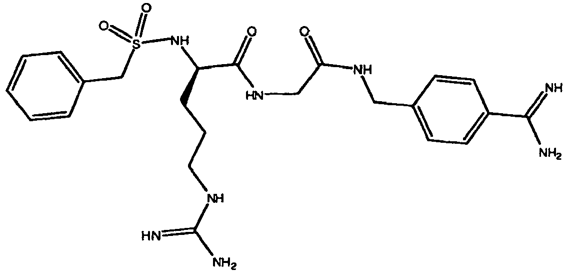

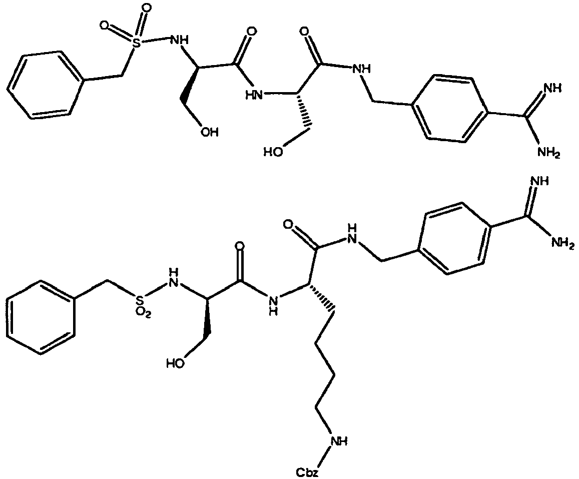

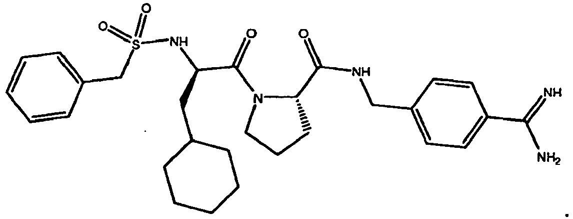

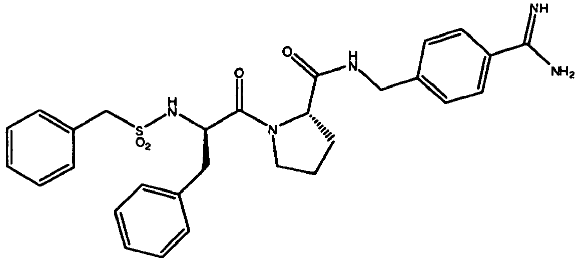

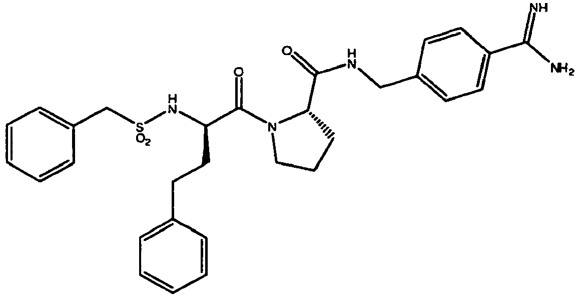

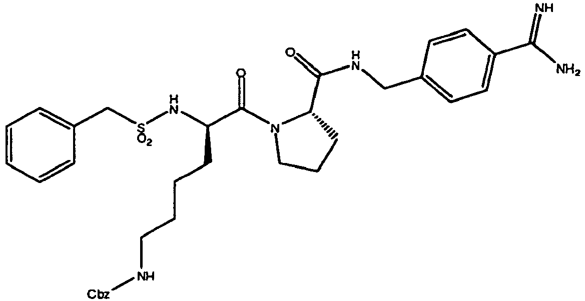

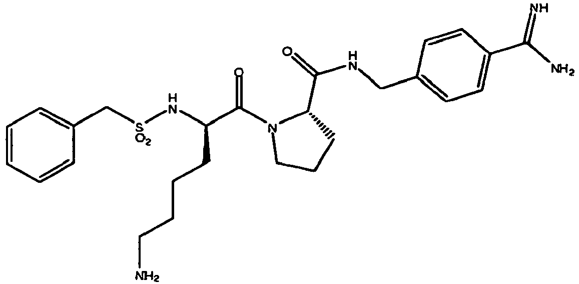

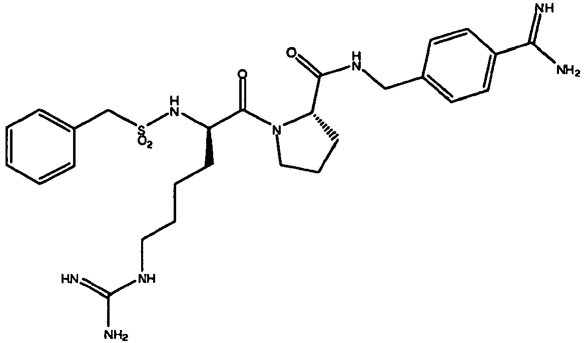

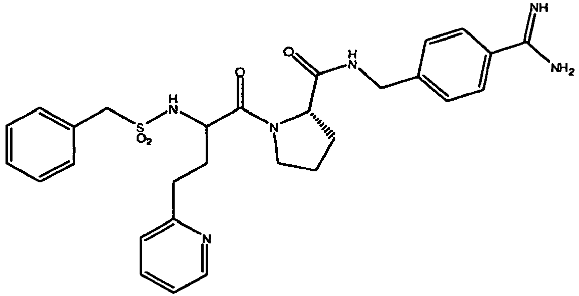

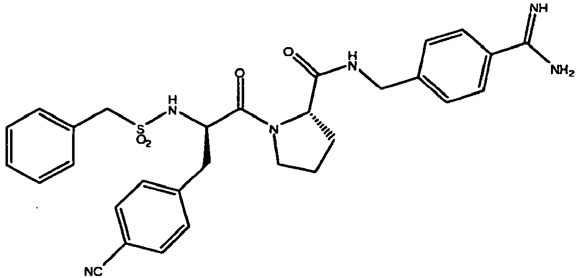

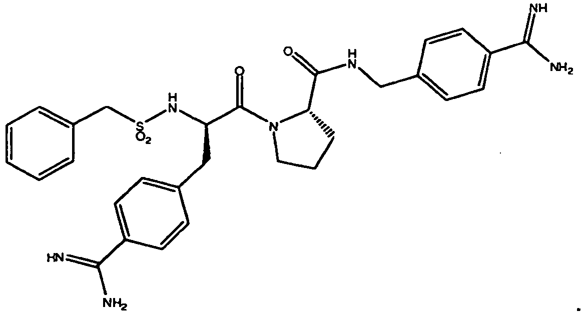

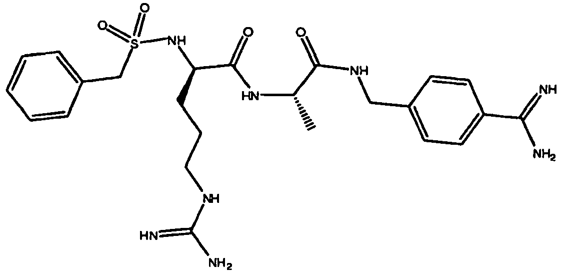

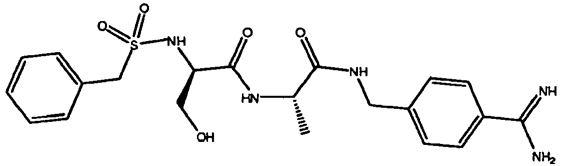

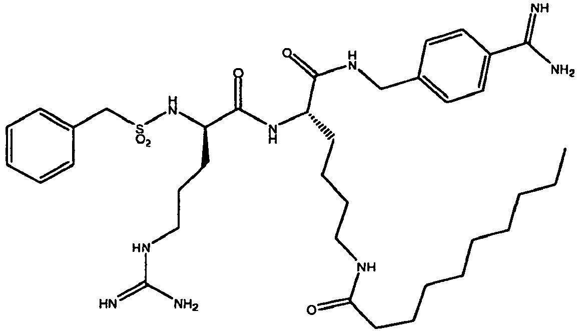

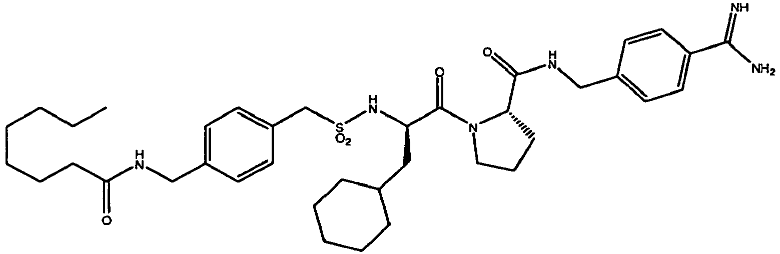

Die erfindungsgemäßen Verbindungen gemäß Formel (I) sowie deren Salze anorganischer und organischer Säuren sind Hemmstoffe der Serinproteasen HAT und TMPRSS2 und können in Form ihrer pharmazeutisch akzeptablen Salze zur Herstellung von Arzneimitteln für Patienten zur Therapie und/oder Prophylaxe von viralen Erkrankungen, beispielsweise von Influenza-Infektionen sowie für die Behandlung von inflammatorischen Atemwegserkrankungen und Krebs verwendet werden. Der Begriff "Patient" bezieht sich dabei gleichermaßen auf Menschen und Wirbeltiere. Damit können die Arzneimittel in der Human- und Veterinärmedizin verwendet werden.

Unter pharmazeutisch akzeptablen Salzen der erfindungsgemäßen Verbindungen zur Verwendung als Hemmstoffe der Serinproteasen HAT und TMPRSS2 gemäß Formel (I) werden die entsprechenden Salze organischer und anorganischer Säuren verstanden, welche nach zuverlässiger medizinischer Beurteilung keine übermäßige Toxizität, Irritationen oder allergische Reaktionen am Patienten auslösen.

Diese pharmazeutisch akzeptablen Salze organischer und anorganischer Säuren sind aufgrund ihrer höheren Wasserlöslichkeit gegenüber den Ausgangs- bzw. Basisverbindungen besonders für die Herstellung von Arzneimitteln geeignet. Diese Salze müssen ein pharmazeutisch verträgliches Anion aufweisen. Geeignete pharmazeutisch verträgliche Säureadditionssalze der erfindungsgemäßen Verbindungen sind z.B. Salze anorganischer Säuren, wie Salzsäure, Bromwasserstoff-, Phosphor-, Metaphosphor-, Salpeter-, Sulfon- und Schwefelsäure oder Salze organischer Säuren wie z.B. Essigsäure, Benzolsulfonsäure, Benzoesäure, Zitronensäure, Ethansulfonsäure, Fumarsäure, Gluconsäure, Glykolsäure, Isethionsäure, Milchsäure, Lactobionsäure, Maleinsäure, Methansulfonsäure, Bernsteinsäure, p-Toluolsulfonsäure, Weinsäure, Trifluoressigsäure.

Der im folgenden verwendete Begriff "physiologisch funktionelles Derivat" bezeichnet jedes physiologisch verträgliche Derivat einer erfindungsgemäßen Verbindung der Formel (I), z. B. einen Ester, der bei Verabreichung an einem Säuger, wie z. B. den Menschen, in der Lage ist, (direkt oder indirekt) eine Verbindung der Formel I oder einen aktiven Metaboliten hiervon zu bilden und für die Verwendung als Hemmstoffe der Serinproteasen HAT und TMPRSS2 geeignet ist. Die Verbindungen gemäß der allgemeinen Formel I können des Weiteren in verschiedenen Formen verwendet werden, z. B. als amorphe und kristalline polymorphe Formen.

Nachfolgend beziehen sich alle Verweise auf die Verwendung von Verbindungen gemäß Formel (I) wie vorstehend beschrieben sowie Salze, Solvate und physiologisch funktionellen Derivate wie hierin beschrieben.

Die Verwendung von Wirkstoffen im Sinne der vorliegenden Erfindung, die sowohl therapeutisch wirksam als auch pharmazeutisch akzeptabel sind, können dem Patienten als Teil einer pharmazeutisch akzeptablen Komposition verabreicht werden. Die Verabreichung kann peroral, parenteral, intravenös, intramuskulär, subkutan, intracisternal, intravaginal, intraperitoneal, intravasculär, intrathekal, intratracheal, intravesikal, topisch, lokal (Puder, Salbe oder Tropfen) oder in Sprayform (Aerosol) erfolgen. Die intravenöse, subkutane, intraperitoneale oder intrathekale Gabe kann dabei kontinuierlich mittels einer Pumpe oder Dosiereinheit erfolgen. Dosierungsformen für die örtliche Administration der erfindungsgemäßen Verbindungen schließen Salben, Puder, Zäpfchen, Sprays und Inhalationsmittel ein. Die aktive Komponente wird dabei unter sterilen Bedingungen mit einem physiologisch akzeptablen Trägerstoff und möglichen Konservierungsmitteln, Puffern, Verdünnungs- und Treibmitteln je nach Bedarf vermischt. Bei der aktiven Komponente kann es sich dabei um Verbindungen gemäß Formel (I), deren pharmazeutisch akzeptable Salze oder um Gemische aus Verbindungen gemäß Formel (I) und deren pharmazeutisch akzeptabler Salze handeln.

### Ausführungsbeispiele

### Methoden zur Analyse der Verbindungen

### Analytische HPLC

Zur analytischen reversed-phase-HPLC wurde eine HPLC-Anlage LC-10A der Firma Shimadzu, bestehend aus den Teilsystemen CTO-10A Säulenofen, LC-10ATvp Pumpen (2 x), DGU-14A Degaser, SIL-10Axl Autoinjektor, SCL-10Avp Systemcontroller, SPD-M10Avp Photodiodenarraydetektor und einer Säule 250/4,6 Nucleodur 100-5 C18 ec der Firma Macherey-Nagel, Germany, unter Benutzung der zugehörigen Software Shimadzu CLASS-VP, Version 7.2.1, verwendet. Die Detektion erfolgte bei 220 nm. Als Elutionsmittel dienten Wasser mit 0,1 % TFA (A) und Acetonitril mit 0,1 % TFA (B), die Analyse erfolgte bei einer Flussrate von 1 ml/min mit einem linearen Gradienten (1 % B/min) und 10 % B als Startbedingung.

### Massenspektrometrie

Die Spektren wurden mit einem Gerät der Firma Applied Biosystems (QTrap 2000) aufgenommen.

### Verwendete Abkürzungen

- Cbz: Benzyloxycarbonyl
- MS: Massenspektrometrie

### Beispiel 1: Synthetisierte Verbindungen

Die für die Experimente verwendeten Verbindungen bzw. Inhibitoren sind bereits bekannt und wurden nach den aus der Literatur bekannten Verfahren synthetisiert und am Ende über präparative HPLC gereinigt und liegen als TFA-Salze vor (Schweinitz et al., J. Biol. Chem. 279, 33613-336122 (2004); Schweinitz et al., Medicinal Chemistry 2, 349-361 (2006), Stürzebecher et al., ChemMedChem 2, 1043-1053 (2007); Hellstern et al., J. Thromb. Haemost. 5, 2119-2126 (2007)).

**Tabelle 1: Übersicht und analytische Charakterisierung der synthetisierten Inhibitoren (n.b. = nicht bestimmt).**

| Nr. | Struktur | MS berechnet MS gef.(M+H)⁺ | HPLC min |
|---|---|---|---|
| 1 | 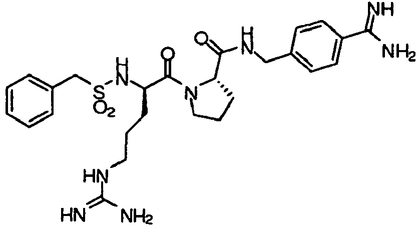 | 556,26 557,0 | 16,80 |
| 2 | 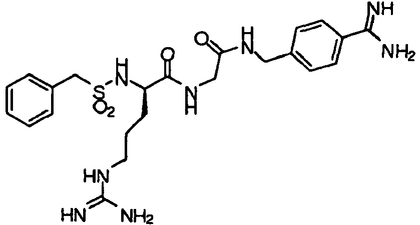 | 516,23 517,3 | 15,35 |
| 3 | 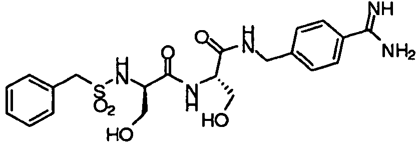 | 477,17 478,2 | 14,76 |
| 4 | 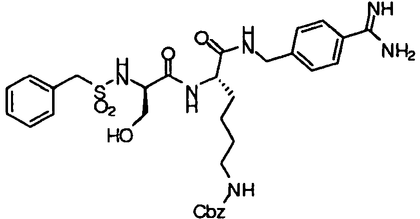 | 652,27 653,3 | 32,74 |
| 5 | 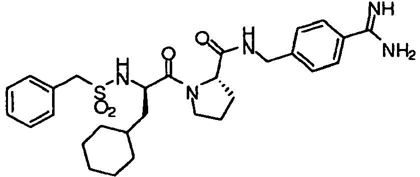 | 553,27 554,2 | 36,93 |
| 6 | 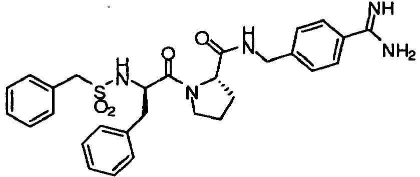 | 547,22 548,1 | 34,17 |
| 7 | 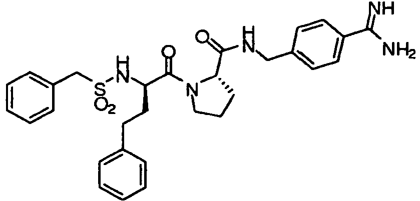 | 561,24 562,1 | 36,15 |
| 8 | 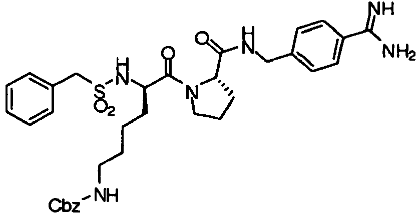 | 662,29 663,3 | 36,93 |
| 9 | 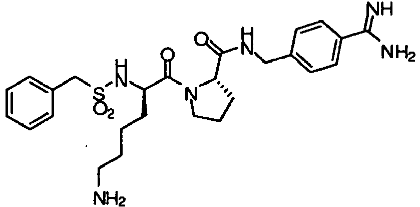 | 528,25 529,2 | 18,16 |
| 10 | 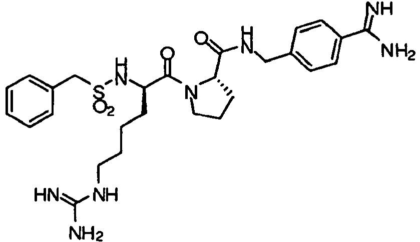 | 570,27 571,2 | 20,01 |
| 11 | 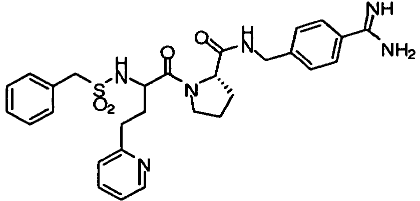 | 562,24 563,1 | 17, 21 /18,11 (Razemat) |
| 12 | 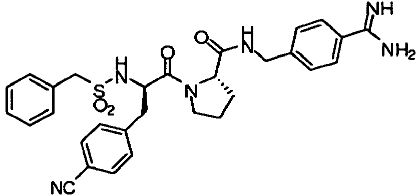 | 572,22 573,0 | 30,82 |
| 13 | 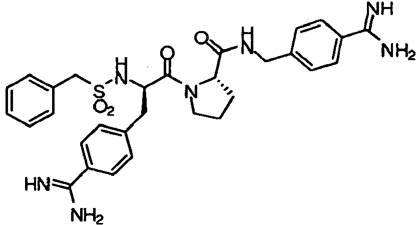 | 589,25 590,16 | n.b. |
| 14 | 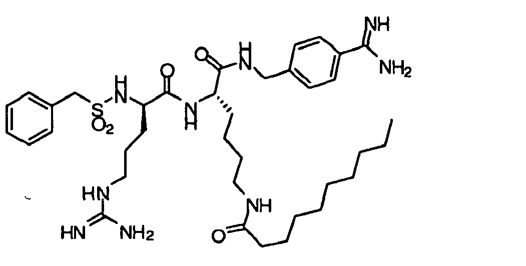 | 741,43 742,3 | n.b. |
| 15 | 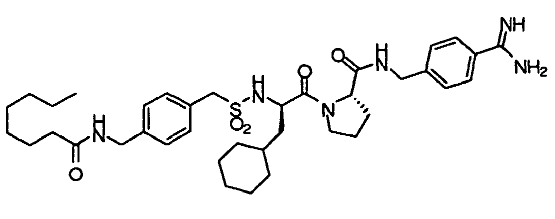 | 708,40 709,3 | n.b. |

**Tabelle 1B: Übersicht und analytische Charakterisierung der synthetisierten Inhibitoren mit den Dissoziationskonstanten Kᵢ (n.b. = nicht bestimmt).**

| Nr. | Struktur | MS berechnet MS gef.(M+H)⁺ | HPLC min | Kᵢ (nM) HAT | Kᵢ (nM) TMPRSS2 |
|---|---|---|---|---|---|
| 1 | 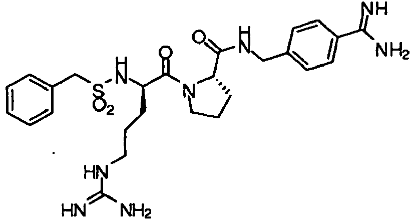 | 556,26 557,0 | 16,80 | 19 | 3 |
| 2 | 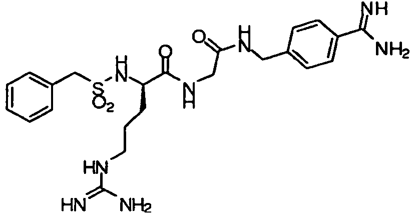 | 516,23 517,3 | 15,35 | 279 | n.b. |
| 3 | 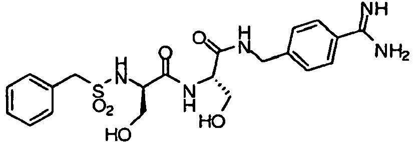 | 477,17 478,2 | 14,76 | 78 | n.b. |
| 4 | 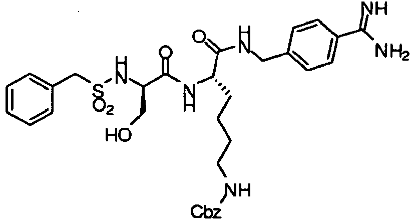 | 652,27 653,3 | 32,74 | 48 | n.b. |
| 5 | 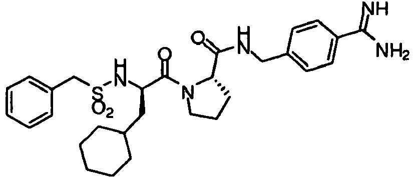 | 553,27 554,2 | 36,93 | 77 | 81 |
| 6 | 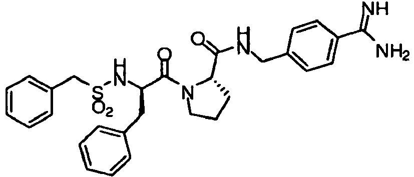 | 547,22 548,1 | 34,17 | 66 | n.b. |
| 7 | 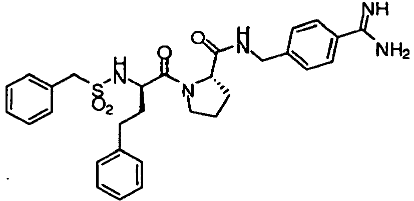 | 561,24 562,1 | 36,15 | 55 | n.b. |
| 8 | 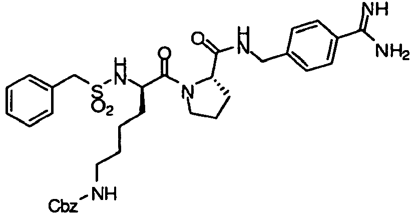 | 662,29 663,3 | 36,93 | 49 | n.b. |
| 9 | 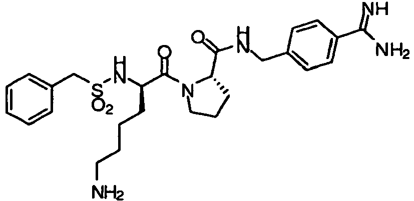 | 528,25 529,2 | 18,16 | 30 | n.b. |
| 10 | 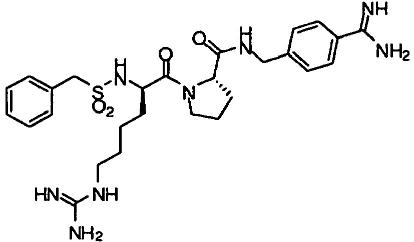 | 570,27 571,2 | 20,01 | 13 | n.b. |
| 11 | 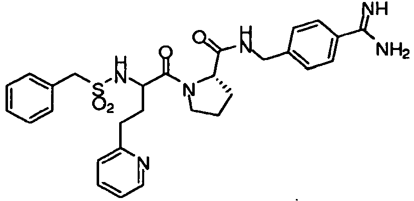 | 562,24 563,1 | 17,21/18,11 (Razemat) | 32 | n.b. |
| 12 | 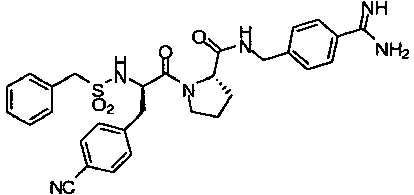 | 572,22 573,0 | 30,82 | 210 | n.b. |
| 13 | 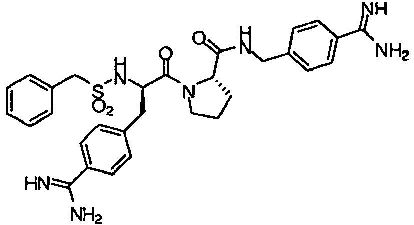 | 589,25 590,16 | n.b. | n.b. | n.b. |
| 14 | 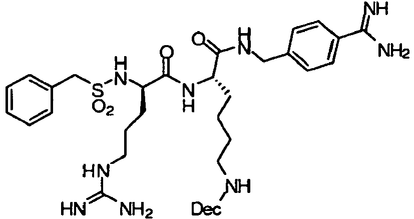 | 741,44 742,4 | 46,6 | 160 | 8 |
| 15 | 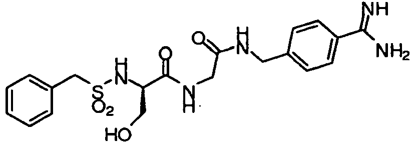 | 447,16 448,3 | 23,52 | 1840 | n.b. |
| 16 | 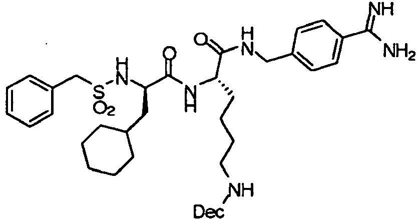 | 739,2 740,1 | 57,7 | >20000 | n.b. |
| 17 | 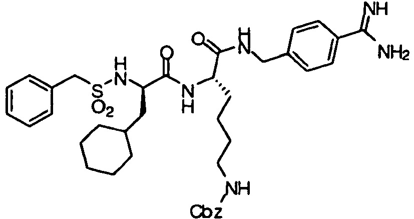 | 718,35 719,3 | 52,1 | 488 | 84 |

### Enzymkinetische Untersuchungen zur Bestimmung der Hemmwirkung auf HAT und TMPRSS2

Die Hemmwirkung der Inhibitoren für HAT (R&D Systems) und TMPRSS2 (katalytische Domäne exprimiert in E. coli) wurden unter Verwendung chromogener Substrate ermittelt. Für HAT wurde d-Cyclohexylalanin-Pro-Arg-7-Aminomethylcoumarin und für TMPRSS2 d-Cyclohexylalanin-Gly-Arg-p-Nitroanilin als Substrat verwendet. Die Bestimmung wurde mit einem Fluoreszenz-Plattenreader Safire² der Firma Tecan (λ_{Ex} = 380 nm, λ_{Em} = 460 nm) für HAT und mit einem UV/Vis Plattenreader iEMS Reader MF 1401 der Firma LABSYSTEMS für TMPRSS2 bei 405 nm durchgeführt. Die Messung erfolgte in Tris-Puffer (50 mM Tris pH 9.5, 0,05% Brij 58 und 1 mg/ml bovines serum Albumin für HAT und 50 mM Tris pH 8.0, 0,154 M NaCl für TMPRSS2) auf Mikrotiter Platten bei Raumtemperatur. Die Inhibitorlösungen geeigneter Konzentration wurden in DMSO hergestellt. Im Messansatz wurden zu 2 µl Inhibitorlösung unterschiedlicher Konzentration jeweils 158 µl Puffer und 20 µl Substratlösung geben, und danach die Reaktion durch Zugabe von 20 µl Enzymlösung gestartet. Jeder Inhibitor wurde mit drei verschiedenen Substratkonzentrationen vermessen (50 µM, 100 µM, 200 µM für HAT und 45 µM, 90 µM und 181 µM für TMPRSS2). Die Inhibitorkonzentration wurde mindestens um den Bereich einer Größenordnung variiert und jeweils die steady-state Geschwindigkeit bestimmt. Die Berechnung der Ki-Werte erfolgte mit Hilfe eines Computerprogramms nach der Methode von Dixon.

### Beispiel 2: Hemmung der HAT-vermittelten Virusvermehrung in Gegenwart synthetischer Serinproteaseinhibitoren

Für die Experimente wurden MDCK-HAT-Zellen mit induzierbarer Expression der Protease HAT verwendet. MDCK-HAT-Zellen wurden durch stabile Transfektion von MDCK-Zellen (Madin Darby Canine Kidney) mit den Plasmiden pcEFTet-On/NEO und pTRE2pur-HAT-FLAG generiert. Die Expression von HAT in diesen Zellen kann durch Zugabe von Doxycyclin zum Kulturmedium induziert werden (Tet-On Expressionssystem, Gossen and Bujard, Science 1995).

Um die Wirksamkeit synthetischer Serinproteaseinhibitoren auf die Hemmung der proteolytischen Aktivierung von Influenzaviren durch HAT zu analysieren, wurde die multizyklische Replikation und Virusausbreitung in MDCK-HAT Zellen in Anwesenheit der Inhibitoren untersucht. Dazu wurden MDCK-HAT-Zellen zunächst in 96-well Platten für 24 h in An- und Abwesenheit von 0.2 µg/ml Doxycyclin kultiviert. Anschließend wurden die Zellen mit dem humanen Influenza-Isolat A/Memphis/14/96 (H1N1) infiziert und für 24 h in An- bzw. Abwesenheit verschiedener Inhibitoren bei 37° C und 5% CO₂ inkubiert. Im Anschluss wurden infizierte Zellen immunohisto-chemisch gegen das virale Nukleoprotein gefärbt. Dabei konnte eine konzentrationsabhängige Hemmung der Virusvermehrung und -Ausbreitung durch die verwendeten synthetischen Inhibitoren gezeigt werden.

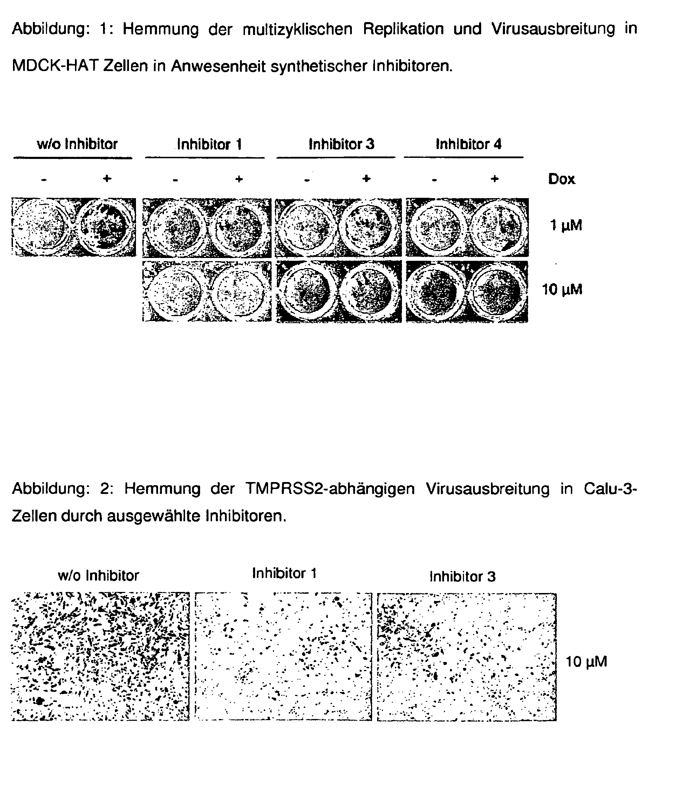

Die Seite 26 enthält nur Beschreibung und keine Abbildung. Die in der ursprünglichen Version der Anmeldeschrift enthaltene Abbildung 1 befindet sich am Ende der vorliegenden Version der Anmeldeschrift.

### Beispiel 3: Hemmung affinitätsgereinigter HAT durch synthetische Serinproteaseinhibitoren

Die in MDCK-HAT-Zellen exprimierte Protease HAT besitzt zusätzlich ein C-terminales Flag-Epitop, welches die Affinitätsreinigung der Protease mit Hilfe von anti-FlagM2-Agarose (Sigma-Aldrich) ermöglicht. HAT-Flag liegt nach der Immunpräzipitation als aktive Protease vor. Die enzymatische Aktivität kann mit Hilfe des Fluoreszenzsubstrats Boc-Leu-Gly-Arg-AMC bestimmt werden.

Für Inhibitionsstudien wurde HAT-Flag zunächst mit Hilfe des *FLAG®Tagged Protein Immunoprecipitation Kits* aus MDCK-HAT-Zellen isoliert. Anschließend wurde die isolierte Protease mit 25 µM Boc-Leu-Gly-Arg-AMC in 50 mM Tris/HCl pH 7,4 in An- oder Abwesenheit der Inhibitoren (Endkonzentrationen 0,1 -10 µM) für 2 h bei 37° C inkubiert. Die Umsetzung des Substrates wurde mit Hilfe eines Fluoreszenz-Spektralphotomers bei 350 nm (Extinktion) sowie 460 nm (Emission) bestimmt. Als Kontrolle diente die Bestimmung der Proteaseaktivität in Abwesenheit eines Inhibitors (entspricht 0% Hemmung).

**Tabelle 2: Hemmwirkung synthetischer Serinproteasehemmstoffe auf die HAT-katalysierte Spaltung des Substrats Boc-Leu-Gly-Arg-AMC.**

| Inhibitorkonzentration (µM) | Prozent Hemmung (%) | | |
|---|---|---|---|
| | Inhibitor 1 | Inhibitor 3 | Inhibitor 5 |
| 0,1 | 22,3 | 29,5 | 27 |
| 1 | 70,7 | 47,1 | 47,4 |
| 10 | 93,1 | 80,3 | 51,5 |

### Beispiel 4: Hemmung der TMPRSS2-vermittelten Virusvermehrung durch synthetische Serinproteaseinhibitoren

Zum Nachweis der Hemmwirkung einer TMPRSS2-vermittelten Virusausbreitung wurden Calu-3-Zellen (humane Atemwegsepithelzellen, endogene Expression von TMPRSS2) verwendet. Dazu wurden die Zellen in 12 Weil-Platten kultiviert und mit dem humanen Influenzavirus-Isolat A/Memphis/14/96 (H1N1) in An- und Abwesenheit verschiedener Inhibitoren für 24 h infiziert. Anschließend wurden infizierte Zellen immunohistochemisch gegen das virale Nukleoprotein gefärbt. Dabei konnte eine konzentrationsabhängige Reduktion der Virusausbreitung in Anwesenheit der verwendeten Inhibitoren 1 und 3 gezeigt werden.

Die Seite 28 enthält nur Beschreibung und keine Abbildung. Die in der ursprünglichen Version der Anmeldeschrift enthaltene Abbildung 2 befindet sich am Ende der vorliegenden Version der Anmeldeschrift.

## Patentansprüche

1. Verbindungen gemäß der allgemeinen Formel (I) 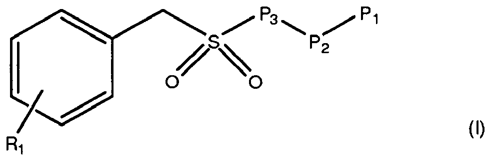 und pharmazeutisch geeignete Salze oder Prodrugs dieser Verbindungen, worin
- R₁ sich in 2-, 3- oder 4-Stellung befindet und ausgewählt ist aus -H oder R₂-CO-NH-CH₂-, worin R₂ eine lineare oder verzweigte Alkylgruppe mit 1 bis 9 Kohlenstoffatomen darstellt; und
- P3 eine einfach oder mehrfach substituierte oder unsubstituierte, natürliche oder unnatürliche α-Aminosäure in der D-Konfiguration ist, nämlich P3 eine Struktur gemäß Formel (II) darstellt 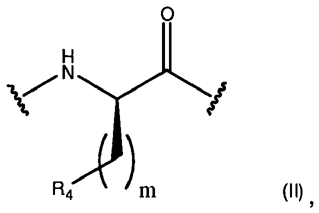 worin
- m = 0, 1, 2, 3, 4 oder 5 ist, und
- R₄ ausgewählt wird aus
• einer unsubstituierten oder gegebenenfalls substituierten Hydroxy-, Amino-, Amid-, -S-CH₃, Amidino-, Guanidino- oder -O-Guanidinogruppe der folgenden Strukturen 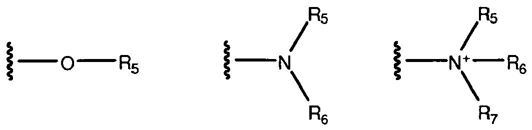 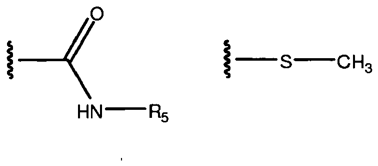 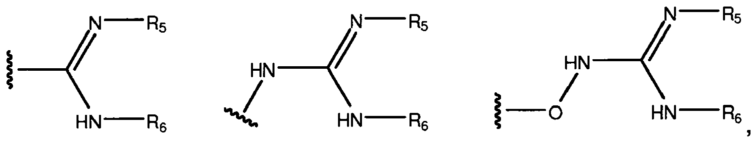 wobei R₅, R₆ und R₇ unabhängig voneinander ein H oder ein Aralkylrest sein können, oder
• aus einem unsubstituierten oder an beliebiger Stelle substituierten Aryl-oder Heteroarylring mit 5 bis 10 Ringatomen, wobei der Heteroarylring bis zu 4 Heteroatome ausgewählt aus N, O oder S enthalten kann, und der gegebenenfalls vorhandene Substituent am Aryl- oder Heteroarylring ausgewählt wird aus Cyano, CF₃, F, Cl, Br, Amino, Aminomethylen, Amidino, Guanidino oder aus folgenden Strukturen 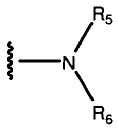 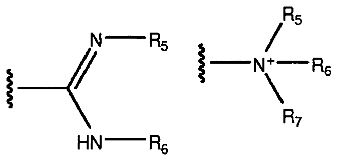 wobei die Reste R₅, R₆ und R₇ wie zuvor definiert sind, oder
• aus einem unsubstituiertem oder an beliebiger Stelle substituierten Cycloalkylring mit 5 bis 7 Ringatomen; und
- P2 eine einfach oder mehrfach substituierte oder unsubstituierte, natürliche oder unnatürliche α-Aminosäure oder α-Iminosäure in der L-Konfiguration ist, nämlich P2 einen a-Aminosäure- oder α-Iminosäurerest gemäß der folgenden Strukturen darstellt 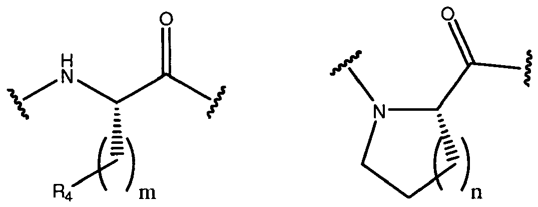
- wobei n gleich 0, 1 oder 2 sein kann, und m und R₄ wie zuvor definiert sind und wenn m gleich 0 oder 1 ist, kann R4 H sein; und
- P1 ausgewählt wird aus folgenden Strukturen 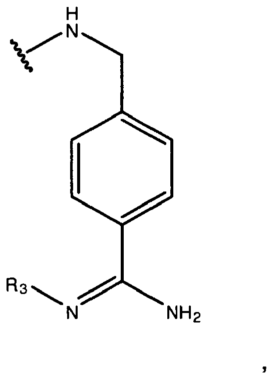 worin R₃ ein H oder im Falle des Prodrugs ein OH, O-CH₃, O-CO-CH₃ oder -CO-O-(CH₂)_{z}-CH₃ und z eine ganze Zahl von 1 bis 5 ist,
zur Verwendung bei der Therapie und/oder Prophylaxe viraler Infektionen und inflammatorischer Atemwegserkrankungen durch Inhibierung der Serinproteasen HAT und TMPRSS2.

2. Die Verbindungen zur Verwendung bei der Therapie und/oder Prophylaxe viraler Infektionen und inflammatorischer Atemwegserkrankungen nach Anspruch 1, **dadurch gekennzeichnet, dass** sich der Rest R₁ in 3- oder 4-Stellung des Phenylrings befindet, bevorzugt ist R₁ ein H.

3. Die Verbindungen zur Verwendung bei der Therapie und/oder Prophylaxe viraler Infektionen und inflammatorischer Atemwegserkrankungen nach einem der Ansprüche 1 oder 2, **dadurch gekennzeichnet, dass** der Rest P3 aus folgenden Strukturen ausgewählt wird 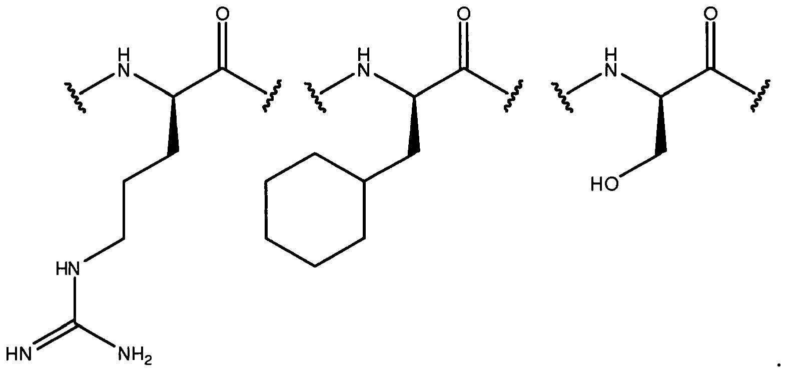 und einen D-Arginin-, D-Cyclohexylalanin- oder D-Serinrest darstellt.

4. Die Verbindungen zur Verwendung bei der Therapie und/oder Prophylaxe viraler Infektionen und inflammatorischer Atemwegserkrankungen nach einem der Ansprüche 1 bis 3, **dadurch gekennzeichnet, dass** der Rest P2 aus folgenden Strukturen ausgewählt wird 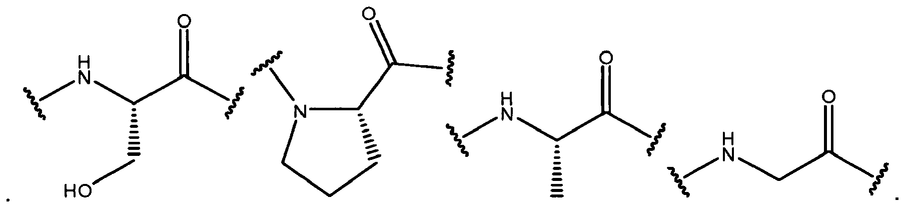 und einen Serin, Prolin- oder Alaninrest in L-Konfiguration oder einen Glycinrest darstellt.

5. Die Verbindungen zur Verwendung bei der Therapie und/oder Prophylaxe viraler Infektionen und inflammatorischer Atemwegserkrankungen nach einem der Ansprüche 1 bis 4, **dadurch gekennzeichnet, dass** es sich bei den Verbindungen gemäß Formel (I) um folgende Strukturen handelt

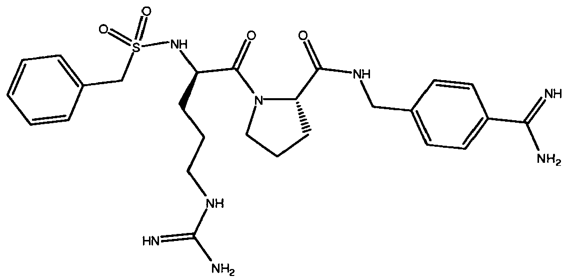

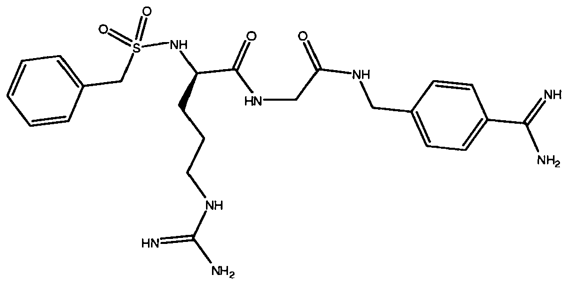

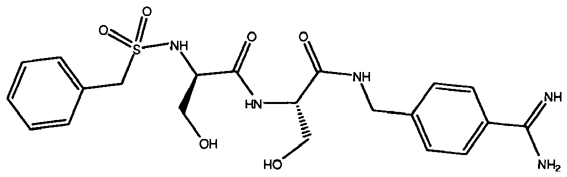

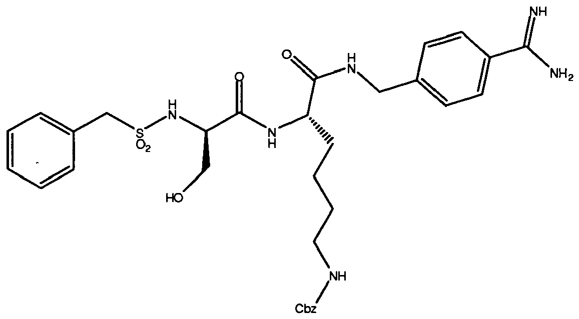

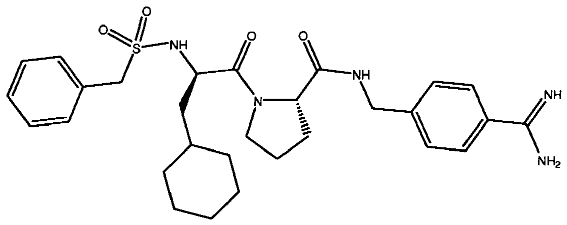

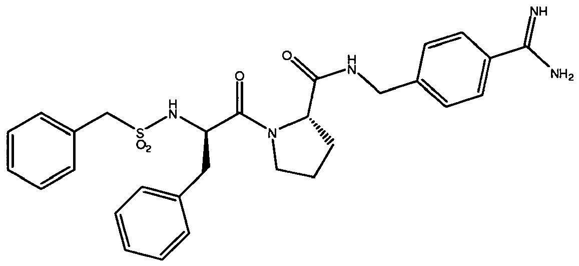

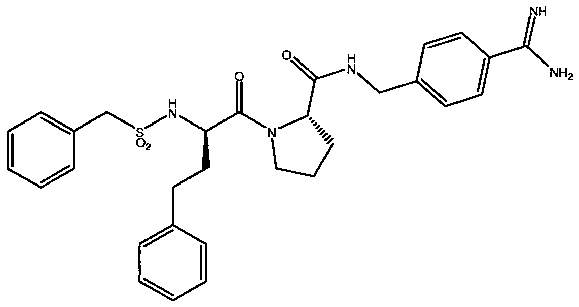

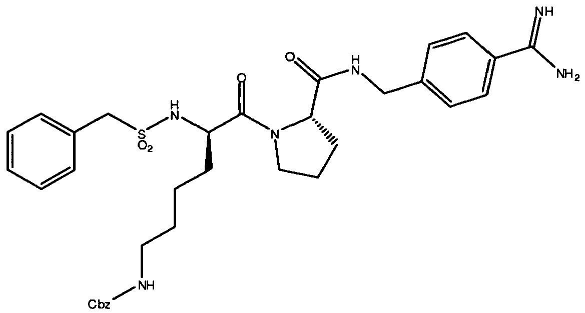

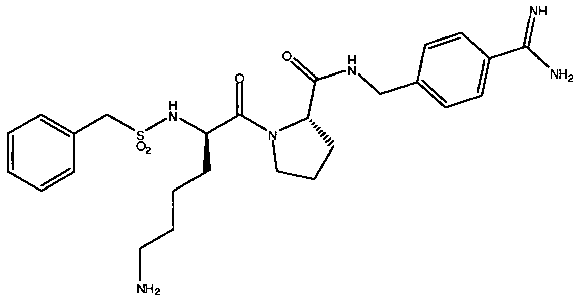

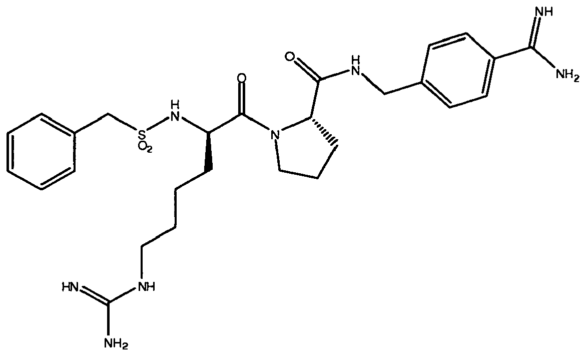

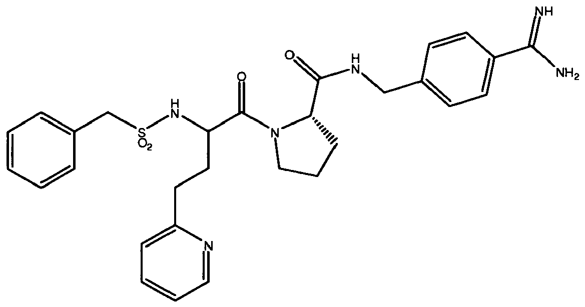

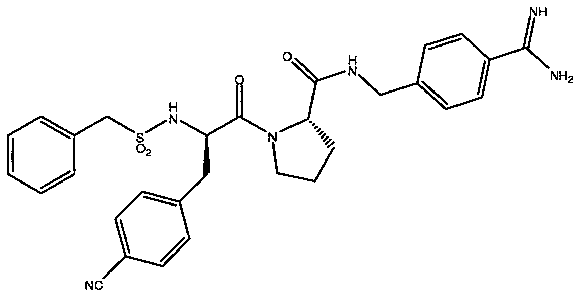

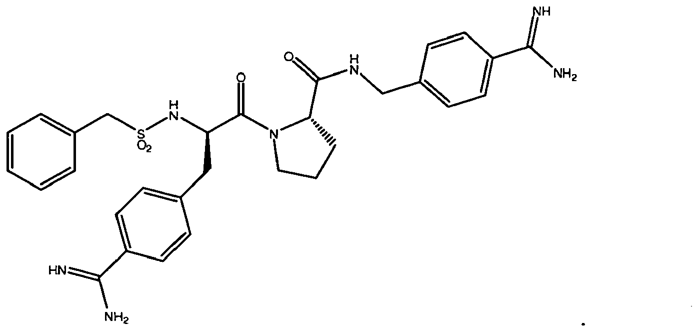

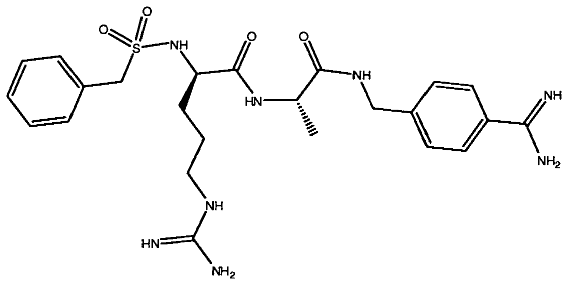

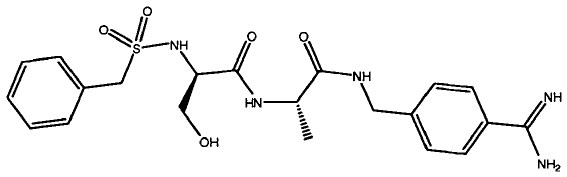

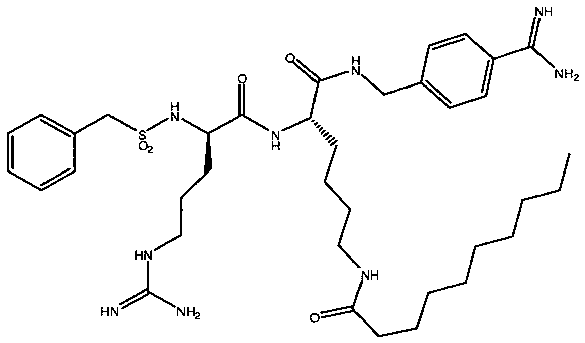

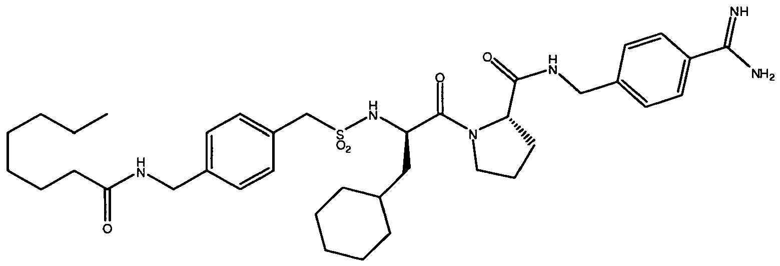

6. Verwendung einer pharmazeutisch akzeptablen Komposition enthaltend Verbindungen gemäß einem der Ansprüche 1 bis 5 und/oder deren pharmazeutisch akzeptable Salze anorganischer und organischer Säuren oder Basen als aktive Komponente und einen physiologisch akzeptablen Trägerstoff zur Herstellung eines Arzneimittels zur Behandlung viraler Infektionen und inflammatorischer Atemwegserkrankungen.

7. Verwendung einer pharmazeutisch akzeptablen Komposition gemäß Anspruch 6, **dadurch gekennzeichnet, dass** sie peroral, parenteral, intravenös, intramuskulär, subkutan, intracisternal, intravaginal, intraperitoneal, intravasculär, intrathekal, intratracheal, topisch, lokal oder als Aerosol verabreicht wird.

8. Verwendung von Verbindungen gemäß einem der Ansprüche 1 bis 5 sowie deren pharmazeutisch akzeptabler Salze anorganischer und organischer Säuren zur Herstellung eines Arzneimittels zur Therapie und/oder Prophylaxe von viralen Erkrankungen, besonders von Influenza-Infektionen.

9. Verwendung von Verbindungen gemäß einem der Ansprüche 1 bis 5 sowie deren pharmazeutisch akzeptabler Salze anorganischer und organischer Säuren zur Herstellung eines Arzneimittels zur Therapie inflammatorischer Atemwegserkrankungen.

## Claims

1. Compounds according to the general formula (I) 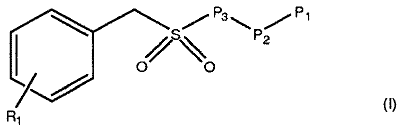 and pharmaceutically acceptable salts or prodrugs of said compounds, in which
- R₁ is in the second, third or fourth position and is selected from -H or R₂-CO-NH-CH₂-, in which R₂ is a linear or branched alkyl group having from 1 to 9 carbon atoms; and
- P3 is a mono- or polysubstituted or unsubstituted, natural or unnatural α-amino acid in D-configuration, that is to say P3 is a structure according to formula (II) 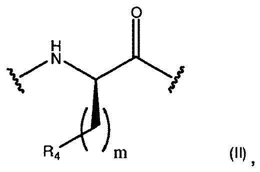
in which
- m = 0, 1, 2, 3, 4 or 5, and
- R₄ is selected from
• an unsubstituted or optionally substituted hydroxyl-, amino-, amide-, -S-CH₃-, amidino-, guanidino- or -O-guanidino group having the following structures 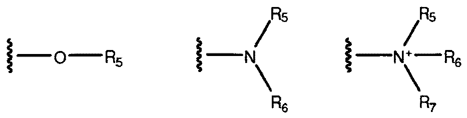 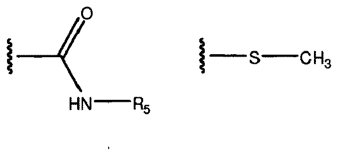 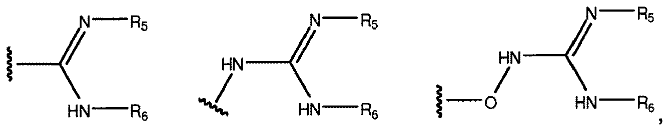 wherein independently of one another R₅, R₆ and R₇ may be a H- or an aralkyl functional group, or
• from an unsubstituted aryl- or heteroaryl ring, or from an aryl- or heteroaryl ring substituted at any position, having from 5 to 10 ring atoms, wherein the heteroaryl ring may contain up to 4 heteroatoms selected from N, O or S, and the optionally existing substituent at the aryl- or heteroaryl ring is selected from cyano-, CF₃-, F-, Cl-, Br-, amino-, aminomethylene-, amidino-, guanidino groups or from the following structures 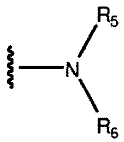 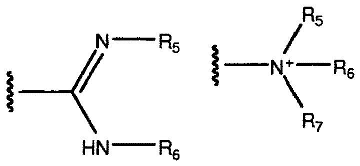 wherein the functional groups R₅, R₆ and R₇ are defined as above, or
• from an unsubstituted cycloalkyl ring, or a cycloalkyl ring substituted at any position, having from 5 to 7 ring atoms; and
- P2 is a mono- or polysubstituted or unsubstituted, natural or unnatural α-amino acid or α-imino acid in L-configuration, that is to say P2 is an α-amino acid group or α-imino acid functional group according to the following structures, 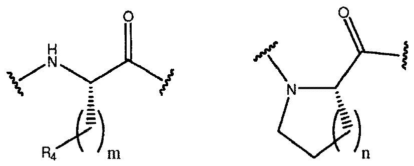
- wherein n may be equivalent to 0, 1 or 2 and m and R₄ are defined as above, and if m is equivalent to 0 or 1, R₄ may be H; and
- P1 is selected from the following structures 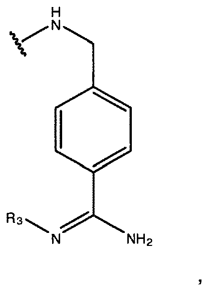 in which R₃ is a H, or in the case of the prodrug is an OH, O-CH₃, O-CO-CH₃ or -CO-O-(CH₂)_{Z}-CH₃ and z is an integer from 1 to 5,
for use in the treatment and/or prevention of viral infections and inflammatory respiratory diseases by inhibiting the serine proteases HAT and TMPRSS2.

2. Compounds for use in the treatment and/or prevention of viral infections and inflammatory respiratory diseases according to claim 1, **characterised in that** the functional group R₁ is in the third position or fourth position of the phenyl ring, R₁ preferably being a H.

3. Compounds for use in the treatment and/or prevention of viral infections and inflammatory respiratory diseases according to either claim 1 or claim 2, **characterised in that** the functional group P3 is selected from the following structures 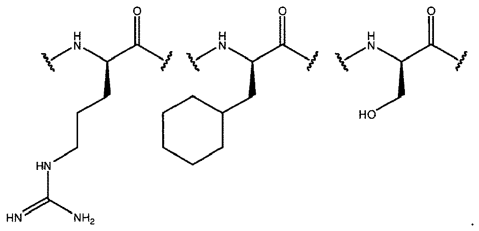 and is a D-arginine- D-cyclohexylalanine- or D-serine functional group.

4. Compounds for use in the treatment and/or prevention of viral infections and inflammatory respiratory diseases according to any of claims 1 to 3, **characterised in that** the functional group P2 is selected from the following structures 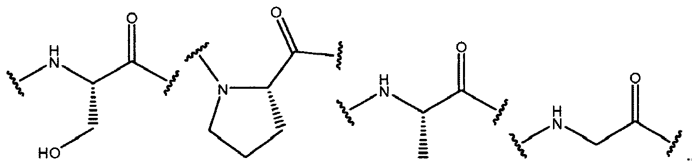 and is a serine, proline, or alanine functional group in L-configuration or is a glycine functional group.

5. Compounds for use in the treatment and/or prevention of viral infections and inflammatory respiratory diseases according to any of claims 1 to 4, **characterised in that** the compounds according to formula (I) have the following structures

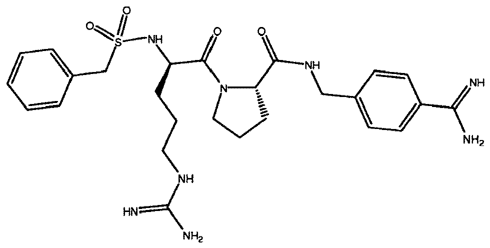

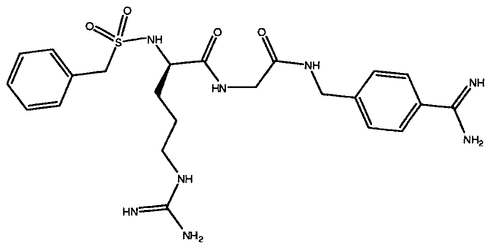

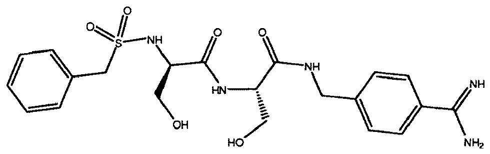

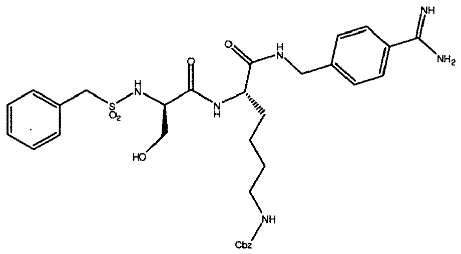

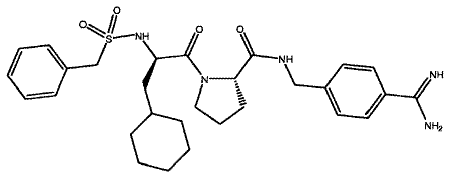

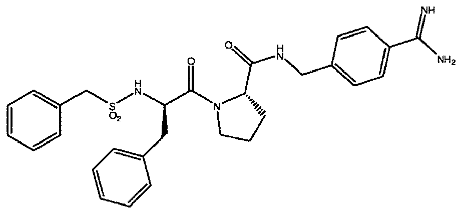

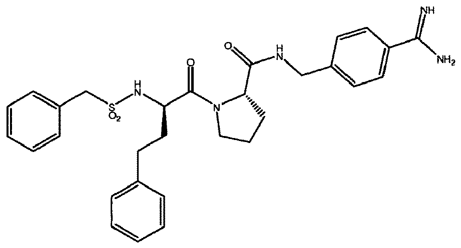

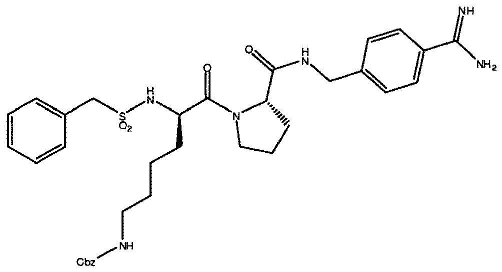

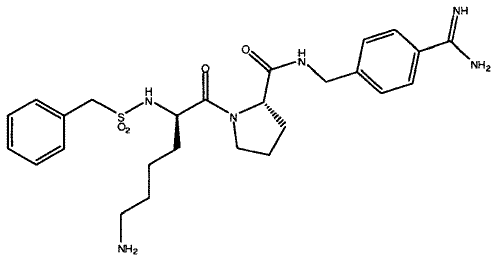

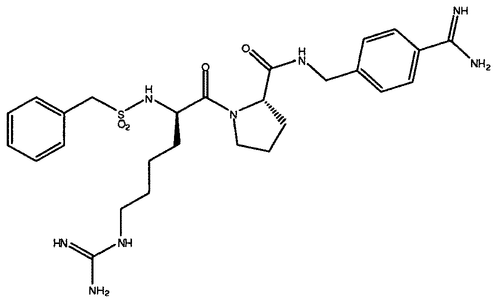

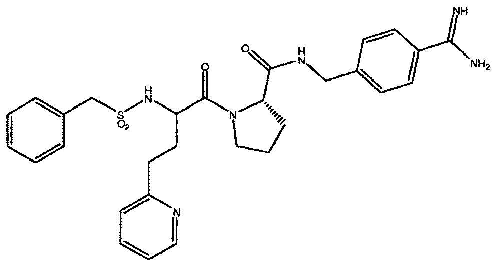

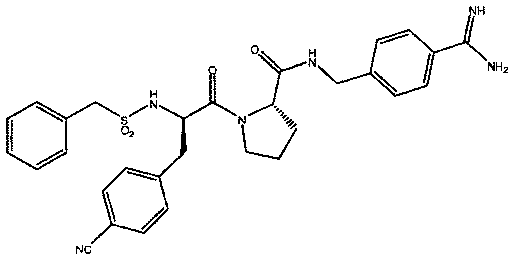

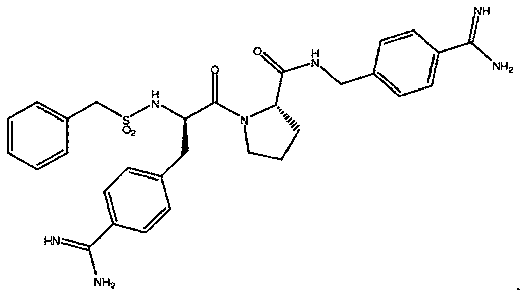

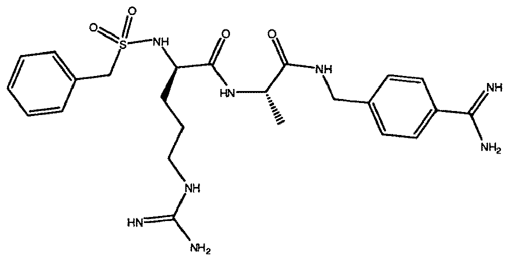

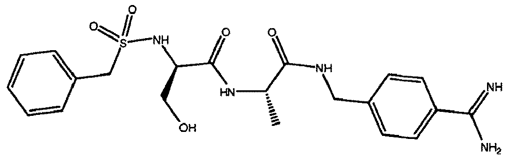

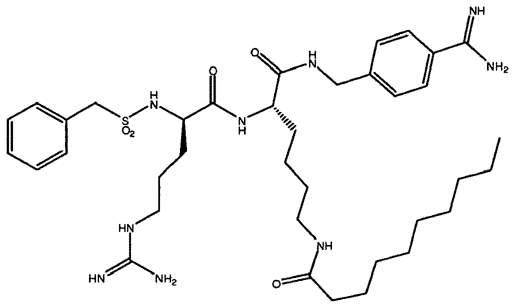

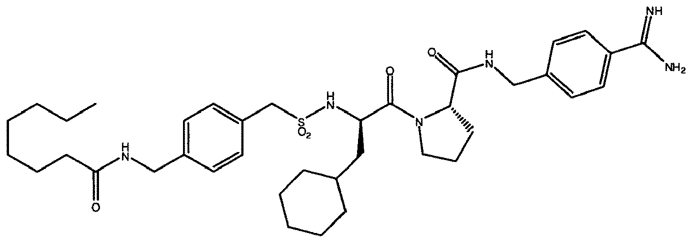

6. Use of a pharmaceutically acceptable composition containing compounds according to any of claims 1 to 5 and/or pharmaceutically acceptable salts thereof of inorganic and organic acids or bases as active components and a physiologically acceptable carrier for preparing a medicine for the treatment of viral infections and inflammatory respiratory diseases.

7. Use of a pharmaceutically acceptable composition according to claim 6, **characterised in that** it is administered perorally, parenterally, intravenously, intramuscularly, subcutaneously, intracisternally, intravaginally, intraperitoneally, intravascularly, intrathecally, intratracheally, topically or locally, or as an aerosol.

8. Use of compositions according to any of claims 1 to 5 and pharmaceutically acceptable salts thereof of inorganic and organic acids for preparing a medicine for the treatment and/or prevention of viral diseases, particularly of influenza infections.

9. Use of compositions according to any of claims 1 to 5 and pharmaceutically acceptable salts thereof of inorganic and organic acids for preparing a medicine for the treatment of inflammatory respiratory diseases.

## Revendications

1. Composés de formule générale (I) : 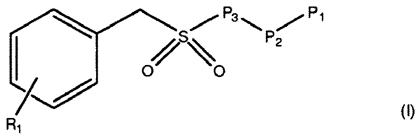 et sels ou promédicaments de ces composés convenant au
plan pharmaceutique,
où :
- R₁ se trouve en position 2, 3 ou 4 et est choisi parmi -H ou R₂-CO-NH-CH₂-, où R₂ représente un groupement alkyle linéaire ou ramifié avec 1 à 9 atomes de carbone ; et
- P₃ est un acide α-aminé naturel ou non, substitué une ou plusieurs fois, en configuration D, en fait P₃ représente une structure de formule (III) : 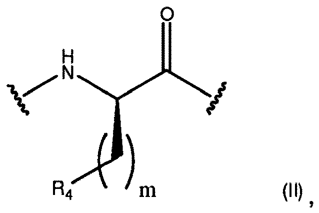
où :
- m = 0, 1, 2, 3, 4 ou 5 et
- R₄ est choisi parmi :
• un groupement hydroxy, amino, amido, -S-CH₃, amidino, guanidino ou -O-guanidino non substitué ou éventuellement substitué de structures suivantes : 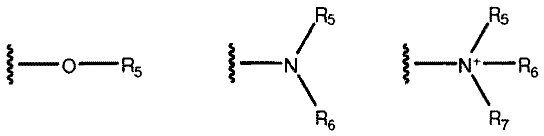 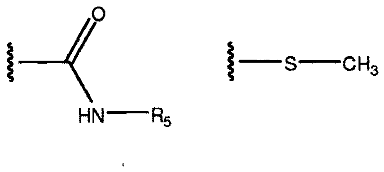 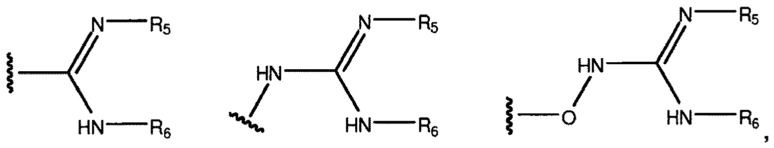 où R₅, R₆ et R₇ peuvent, indépendamment l'un de
l'autre, être H ou un groupement aralkyle, ou
• un cycle aryle ou hétéroaryle non substitué ou substitué en un point quelconque avec 5 à 10 atomes cycliques, où le cycle hétéroaryle peut contenir jusqu'à 4 hétéroatomes choisis parmi N, 0 ou S, et le substituant éventuellement présent sur le cycle aryle ou hétéroaryle est choisi parmi les groupements cyano, CF₃, F, Cl, Br, amino, aminométhylène, amidino, guanidino ou les structures suivantes : 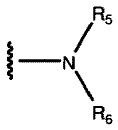 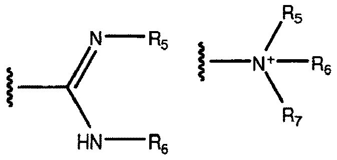 où les radicaux R₅, R₆ et R₇ sont tels que définis ci-dessus ou
• un cycle cycloalkyle non substitué ou substitué en un point quelconque avec 5 à 7 atomes cycliques ; et
- P₂ est un acide α-amino ou un acide α-imino naturel ou pas, substitué une ou plusieurs fois ou non substitué en configuration L, en fait P₂ représente un acide α-amino ou un acide α-imino selon les structures suivantes : 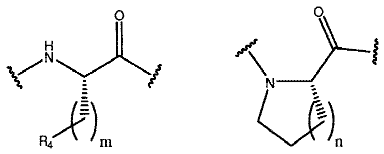
- où n peut être égal à 0, 1 ou 2 et m et R₄ sont tels que définis ci-dessus et, lorsque m est égal à 0 ou à 1, R₄ peut être H ; et
- P₁ est choisi parmi les structures suivantes : 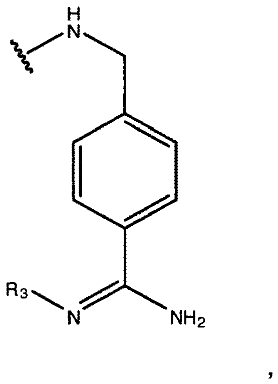
où R₃ est H ou, dans le cas du promédicament, OH, O-CH₃, O-CO-CH₃ ou -CO-O-(CH₂)_{z}-CH₃ et z est un nombre entier de 1 à 5,
pour utilisation en thérapie et/ou en prophylaxie
d'infections virales et de maladies inflammatoires des voies respiratoires par inhibition des sérine protéases HAT et TMPRSS2.

2. Composés pour utilisation en thérapie et/ou en prophylaxie d'infections virales et de maladies inflammatoires des voies respiratoires selon la revendication 1, **caractérisés en ce que** le radical R₁ se trouve en position 3 ou 4 du cycle phényle, de préférence R₁ est H.

3. Composés pour utilisation en thérapie et/ou en prophylaxie d'infections virales et de maladies inflammatoires des voies respiratoires selon l'une quelconque des revendications 1 ou 2, **caractérisés en ce que** le radical R₃ est choisi parmi les structures suivantes : 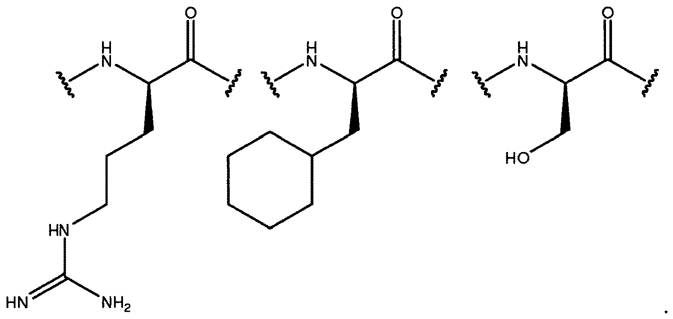 et un radical D-arginine, D-cyclohexylalanine ou D-sérine.

4. Composés pour utilisation en thérapie et/ou en prophylaxie d'infections virales et de maladies inflammatoires des voies respiratoires selon l'une quelconque des revendications 1 à 3, **caractérisés en ce que** le radical P₂ est choisi parmi les structures suivantes : 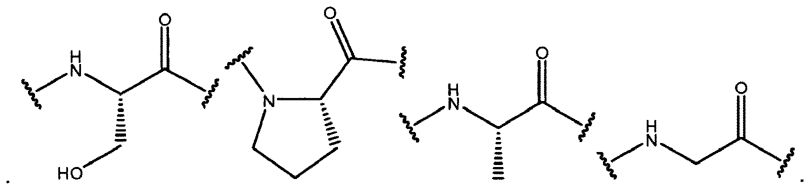 et un radical sérine, proline ou alanine en
configuration L ou un radical glycine.

5. Composés pour utilisation en thérapie et/ou en prophylaxie d'infections virales et de maladies inflammatoires des voies respiratoires selon l'une quelconque des revendications 1 à 4, **caractérisés en ce que** les composés de formule (I) ont les structures suivantes :

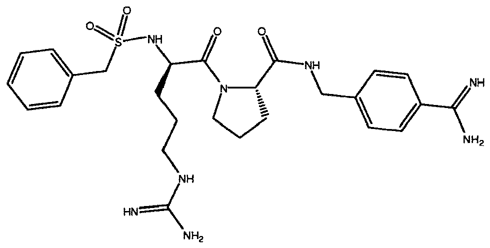

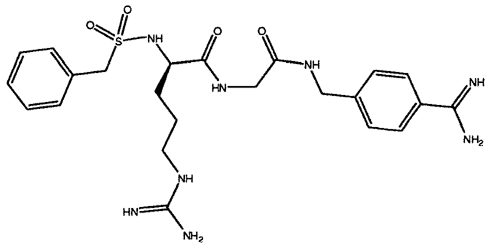

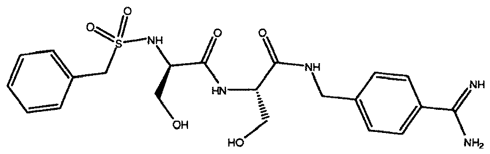

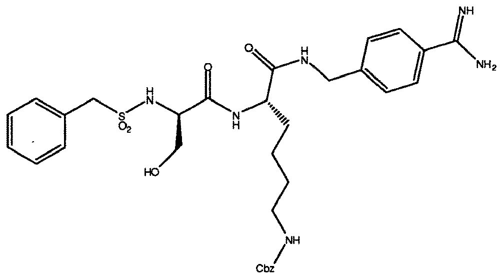

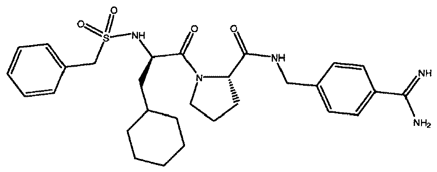

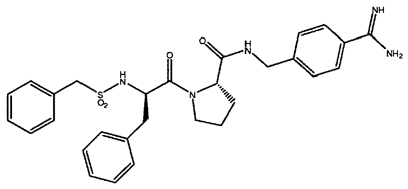

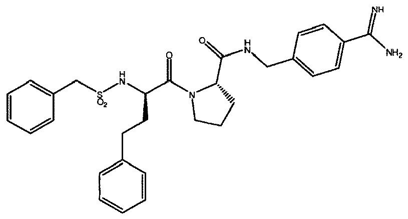

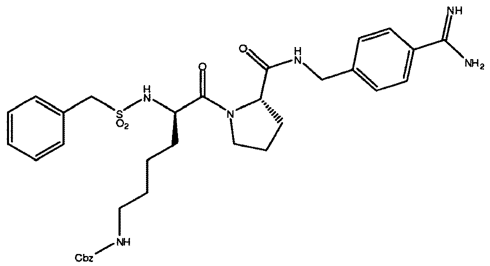

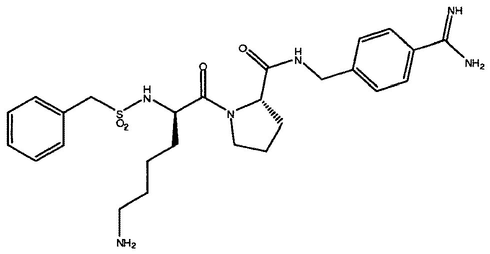

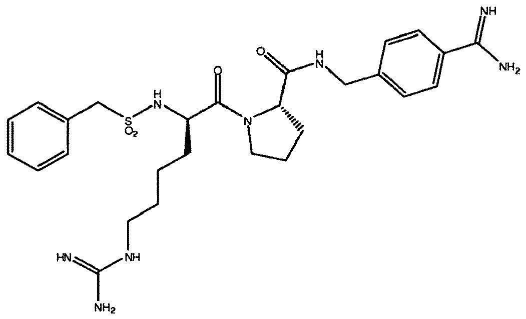

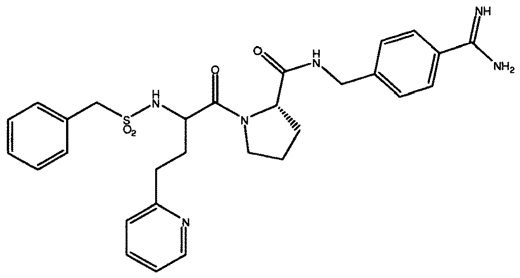

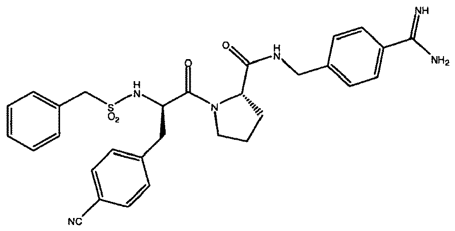

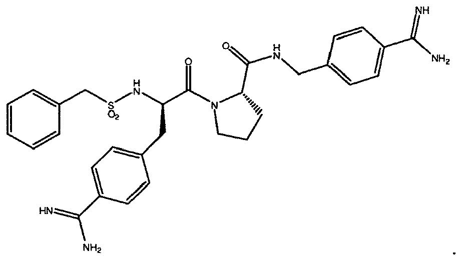

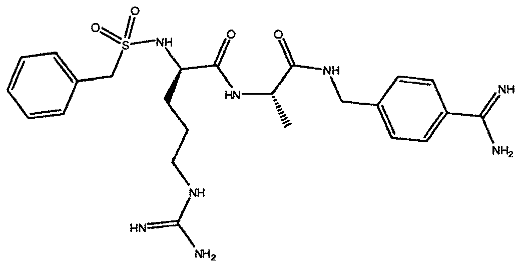

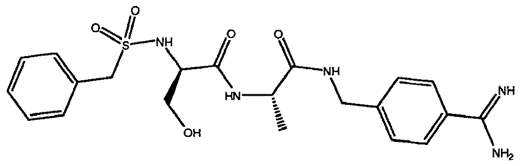

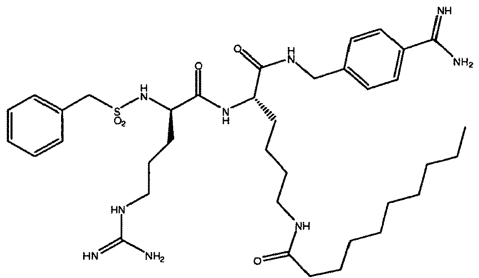

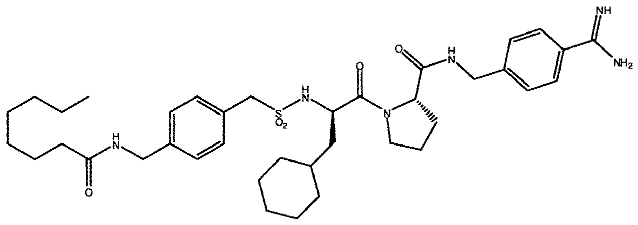

6. Utilisation d'une composition pharmaceutiquement acceptable contenant des composés selon l'une quelconque des revendications 1 à 5 et/ou leurs sels pharmaceutiquement acceptables d'acides ou de bases inorganiques et organiques comme composants actifs et un véhicule physiologiquement acceptable pour la fabrication d'un médicament pour le traitement d'infections virales et de maladies inflammatoires des voies respiratoires.

7. Utilisation d'une composition pharmaceutiquement acceptable selon la revendication 6, **caractérisée en ce qu'**elle est administrée par voie perorale, parentérale, intraveineuse, intramusculaire, sous-cutanée, intracisternale, intravaginale, intrapéritonéale, intravasculaire, intrathécale, intratrachéale, topique, locale ou par aérosol.

8. Utilisation de composés selon l'une quelconque des revendications 1 à 5, ainsi que de leurs sels pharmaceutiquement acceptables d'acides inorganiques et organiques pour la fabrication d'un médicament pour la thérapie et/ou la prophylaxie de maladies virales, en particulier d'infections dues à l'influenza.

9. Utilisation de composés selon l'une quelconque des revendications 1 à 5, ainsi que de leurs sels pharmaceutiquement acceptables d'acides inorganiques et organiques pour la fabrication d'un médicament pour la thérapie de maladies inflammatoires des voies respiratoires.
